(19) Europäisches Patentamt
European Patent Office
Office européen des brevets

(11) Veröffentlichungsnummer: **0 141 178 B1**

# (12) EUROPÄISCHE PATENTSCHRIFT

(45) Veröffentlichungstag der Patentschrift: **26.06.91**

(51) Int. Cl.⁵: **C12M 1/40**, C12Q 1/26

(21) Anmeldenummer: **84110768.3**

(22) Anmeldetag: **10.09.84**

## (54) Anordnung zum Messen der Konzentration eines Stoffes.

(30) Priorität: **10.09.83 DE 3332745**

(43) Veröffentlichungstag der Anmeldung:
**15.05.85 Patentblatt 85/20**

(45) Bekanntmachung des Hinweises auf die Patenterteilung:
**26.06.91 Patentblatt 91/26**

(84) Benannte Vertragsstaaten:
**AT CH DE FR GB IT LI NL SE**

(56) Entgegenhaltungen:
**DE-A- 1 932 581**
**GB-A- 2 000 595**

**TRAC TRENDS IN ANALYTICAL CHEMISTRY, Band 1, Nr. 8, 1982, Seiten 175-179, Cambridge, GB; C.R. MARTIN: "Current trends in ion-selective electrodes"**

(73) Patentinhaber: **Kessler, Manfred, Prof. Dr. med.**
**An der Hornwiesen 50**
**W-8520 Erlangen(DE)**

Patentinhaber: **Höper, Jens**
**Moorbachweg 28**
**W-8520 Erlangen(DE)**

(72) Erfinder: **Kessler, Manfred, Prof. Dr. med.**
**An der Hornwiesen 50**
**W-8520 Erlangen(DE)**
Erfinder: **Höper, Jens**
**Moorbachweg 28**
**W-8520 Erlangen(DE)**

(74) Vertreter: **von Willich, Werner, Dipl.-Phys.**
**Robert-Koch-Strasse 20**
**W-8000 München 22(DE)**

**EP 0 141 178 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Anordnung zum Messen der Konzentration eines Stoffes nach dem Oberbegriff des Anspruchs 1. Eine solche gattungsmäßige Anordnung ist aus der DE-PS 27 30 143 bekannt. Die bekannte Anordnung ist für Konzentrationsmessungen insbesondere deswegen besonders Vorteilhaft, weil durch das Vorsehen einer (Schutz-)Membran an der Elektrode größere (und damit billigere, einfacher herzustellende und zu handhabende, geringere Impedanz aufweisende) Elektroden verwendbar sind, ohne daß Probleme durch den Diffusionsgradienten, die sonst bei größeren Elektroden auftreten, entstehen und von der Elektrode schädliche, nicht zur Meßreaktion beitragende Stoffe ferngehalten werden können. Es ist daher wünschenswert, die gattungsgemäße Anordnung derart weiterzubilden, daß auch die Konzentration anderer wichtiger, insbesondere physiologisch wichtiger, Stoffe gemessen werden kann. Dies wird erfindungsgemäß durch die Weiterbildung nach dem Kennzeichen des Anspruchs 1 erreicht.

Daß der zu messende Stoff im Elektrolytraum vorliegt, bedeutet insbesondere, daß er dorthin aus einem Medium, in dem seine Konzentration letztlich bestimmt werden soll, diffusibel ist oder im Elektrolytraum entsteht, wie im Rahmen der Erfindung unten noch erläutert wird. Daß die Meßelektrode als Anode ausgebildet ist, bedeutet insbesondere, daß sie durch die Polarisationsspannung oder die geeignete Wahl der Referenzelektrode als Anode wirkt.

Unter Grundmembran wird die (bevorzugt aus PVC oder Silikonkautschuk bestehende) Membran vor der Hinzufügung der lipophilen Ionen und/oder der Carrier verstanden, während die Gesamtmembran die Membran nach der Hinzufügung ist.

Durch diese Weiterbildung ist es möglich, die Konzentration des als physiologisches "Abfallprodukt" besonders wichtigen $H_2O_2$ auf einfache Weise, ohne Drifterscheinungen und ohne Fehler durch einen Diffusionsgradienten, genau zu messen. Dabei war es überraschend, daß eine Protonenundurchlässigkeit die Messung von $H_2O_2$ ermöglichen würde. Die Reaktionen, die bei der Oxidation des $H_2O_2$ bei dieser Anordnung ablaufen, sind auch im einzelnen noch nicht geklärt.

In einer besonders vorteilhaften und ein günstiges Reaktionsverhalten bewirkenden Weiterbildung sind die Ionen Anionen. Mit dem besonders bevorzugten Hexa-Decyl-Pyridinium-Chlorid wird eine große Steilheit erreicht. Es wird vermutet, daß die Anionen vorteilhaft zum Entstehen einer Polarisationsschicht an der Grenze Elektroden/Schutzmembran beitragen können. Auch die carriergebundenen Ionen können Anionen sein und sind dann auch im Elektrolyten enthalten.

Besonders vorteilhaft sind die carriergebundenen Ionen Kationen, insbesondere Kaliumionen. Kaliumionen sind vor allem deswegen besonders günstig, weil der Kaliumgehalt physiologischer Flüssigkeiten weitestgehend konstant ist und somit dann, wenn die Anordnung mit solchen Flüssigkeiten zur Messung des Partialdruckes in Verbindung gebracht wird, überschaubare Diffusionsverhältnisse herrschen bzw. der Elektrolyt, der die carriergebundenen Ionen ebenfalls enthält, geeignet eingestellt werden kann, weil der Kaliumgehalt oft vorbekannt ist. Es entstehen also keine osmotischen Probleme.

Besonders vorteilhaft ist der Carrier Valinomycin. Dieser Carrier hat sich in der Praxis bewährt. Ferner ist er ein besonders an das Kalium angepaßter Carrier.

Bevorzugt ist der Carrier ein Kationencarrier von hoher Mobilität und Spezifität, besonders für Protonen, bevorzugt Tri-n-dodecylamin. Mit einem Kationencarrier vom Typ des Tri-n-dodecylamins, der bevorzugt Protonen komplexiert, lassen sich sehr schnelle Ansprech- und Abklingzeiten des Meßwertes erreichen.

Unter Protonenimpermeabilität wird also die (praktische) Protonenimpermeabilität der Grundmembran (z.B. aus PVC) ohne Zusatzstoffe verstanden.

Je nach Art der Zusatzstoffe ergibt sich ein unterschiedliches Verhalten der Gesamtmembran.

1. Die Membran enthält (nur) lipophile Ionen, z.B. das als Puffer (Protonenakzeptor) wirkende Hexa-Decyl-Pyridinium-Chlorid. Hier ist dann auch die Gesamtmembran praktisch protonenimpermeabel, d. h. innerhalb der Grenzen einer $H^+$-Ionenpermeabilität, die für eine Off-Reaktion einer Elektrode mit schneller Abklingzeit zu fordern wäre, ist auch die PVC-Gesamtmembran mit Hexa-Decyl-Pyridinium-Chlorid praktisch protonenimpermeabel.
Es ergibt sich ein relativ rascher Anstieg auf den Meßwert, und dieser wird für mehrere Stunden, größenordnungsmäßig bis zu 12 Stunden, gehalten. Eine solche Ausbildung ist vor allem für die Einmalmessung durch Einmal-Einstich-Elektroden, welche Messungen durch ein nachfolgendes System verarbeitet werden sollen, vorteilhaft, da der Meßwert länger ansteht. Auch durch ihre geringen Kosten ist eine solche Membran besonders vorteilhaft.

2. Die Membran enthält Carrier, wie z. B. Valinomycin, die durch ihre, wenn auch geringe, Querempfindlichkeit für Protonen diese befördern. Die $H^+$-Komplexierung erfolgt hier an der Elektrodengrenzfläche zwar relativ rasch, aber, da praktisch kein Eimerkettenphänomen auftritt, wird die Protonenabgabe verzögert. Dann erhält man ebenfalls eine relativ schnelle Anstiegszeit

auf den Meßwert, aber auch eine etwas schnellere Abklingzeit (z. B. zwei Stunden) als im Falle bspw. des Hexa-Decyl-Pyridinium-Chlorids. Solche Gesamtmembranen ergeben Anordnungen, die den Meßwert für die Verarbeitung ebenfalls länger halten, aber im Laufe des Tages auch mehrfach verwendbar sind.

3. Die Membran enthält einen Protonencarrier von hoher Mobilität. Dann ergeben sich schnelle Anstiegs- und Abklingzeiten, was für fortlaufende Messungen günstig ist.

Besonders bevorzugt ist eine Polarisationsspannungsquelle vorgesehen, deren Pluspol an die Meßelektrode angeschlossen ist. Es hat sich herausgestellt, daß die Anordnung besonders günstig mit einer Polarisationsspannung betrieben wird. Allerdings ist es auch möglich, diese fortzulassen. Dann muß durch die Wahl der Referenzelektrode sichergestellt sein, daß die Meßelektrode die Anode ist.

Besonders bevorzugt ist die Abschlußmembran für $H_2O_2$ durchlässig. Dadurch ist eine Diffusion des zu messenden Stoffes in den Elektrolytraum, mit dem sich ein Gleichgewicht einstellt, das dann seinerseits durch die Anordnung über das an der Meßelektrode oxidierte $H_2O_2$ gemessen wird, möglich. Hierfür hat sich Polytetrafluoräthylen besonders bewährt.

In einer besonders vorteilhaften Weiterbildung ist der Elektrolytraum auch als Reaktionsraum ausgebildet, in dem sich ein Enzym befindet, das eine durch die Abschlußmembran in den Elektrolyten diffusible Substanz unter Bildung von $H_2O_2$ umsetzt. Durch diese Anordnung wird es in sehr einfacher und vorteilhafter Weise möglich, die Konzentration insbesondere wichtiger physiologischer Substanzen zu messen. Dadurch, daß die Substanz durch die Abschlußmembran in den Elektrolyten diffusibel ist, die Abschlußmembran also für diese Substanz durchlässig ist, bildet sich dann, wenn die Anordnung über die Abschlußmembran mit einer die zu messende Substanz enthaltenden Flüssigkeit, z. B. Körperflüssigkeit, in vivo oder in vitro, in Berührung gebracht wird, ein Gleichgewicht zwischen der Substanz in der zu messenden Flüssigkeit und im Elektrolyten aus. Durch die Wahl eines geeigneten Enzyms wird aus der Substanz $H_2O_2$ gebildet, dessen Konzentration dann wiederum mit Hilfe der membranbedeckten Meßelektrode gemessen wird, wodurch ein Maß für die Konzentration der Substanz in der zu messenden Flüssigkeit gewonnen wird. Die Abschlußmembranen sind in diesem Falle Porenmembranen.

Dabei liegt das Enzym bevorzugt in wässriger Lösung vor. Es kann aber alternativ bevorzugt strukturgebunden vorliegen.

Bevorzugt ist die Abschlußmembran für kleinere Moleküle durchlässig, für größere Moleküle hingegen undurchlässig. Sie ist also eine Porenmembran. Dadurch wird verhindert, daß das Enzym aus dem Elektrolytraum/Reaktionsraum in einen zu messenden Raum diffundiert oder störende andere Enzyme, die für die Umsetzung anderer Substanzen zuständig sind, in den Elektrolytraum geraten. Unter "kleinere Moleküle" sollen hier Molküle bis zu einem Molekulargewicht von ca. $10^4$ Dalton, unter "größere Moleküle" solche von einem Molekulargewicht größer als $10^4$ Dalton verstanden werden. Im Einzelfall wird die Porengröße entsprechend zu wählen sein, um erwünschte Substanzen (noch) durchzulassen und unerwünschte nicht (mehr) durchzulassen.

Bevorzugt ist das Enzym Glucose-Oxidase und ist die Abschlußmembran für Glucose und Gluconsäurelacton durchlässig. Die Messung des Glucosegehaltes im Blut oder anderen Körperflüssigkeiten ist angesichts der großen und wachsenden Zahl von Diabetikern von außergewöhnlicher Bedeutung. Durch die angegebenen Merkmale liegt eine Vorrichtung vor, die die Glucose-Konzentration in Flüssigkeiten mit bisher nicht möglicher Einfachheit und Schnelligkeit und dabei großer Genauigkeit ohne die Notwendigkeit einer Nacheichung in kurzen Abständen (bisher oft sogar Minutenabstand) zu bestimmen gestattet.

Die eben geschilderten Vorzüge lassen sich besonders günstig ausnützen, wenn die Anordnung, ggfs. und bevorzugt einschließlich der Polarisationsspannungsquelle, als integrierte, implantierbare Einheit ausgebildet ist und einen Geber oder Sender für das Meßergebnis aufweist. Eine solche Anordnung kann nämlich in besonders vorteilhafter Weise mit den kürzlich entwickelten implantierbaren Dosierpumpen für Insulin zusammen-wirken. Ferner kann dem Träger auch ein direktes Warnsignal gegeben werden, das ihm anzeigt, ob der zuträgliche Bereich der Glucose-Konzentration im Blut unter- bzw. überschritten wird.

Besonders bevorzugt ist die Bezugselektrode eine Edelmetallelektrode, die als Kathode geschaltet ist und die mit einer Membran bedeckt ist, die vorzugsweise einen Protonencarrier, besonders bevorzugt Tri-n-dodecylamin, enthält. Dadurch läßt sich die Genauigkeit der Messung weiter verbessern.

Als besonderer Vorteil der Erfindung ist noch anzumerken, daß der erzielte Meßwert, also das erhaltene Signal, ca. das Doppelte dessen beträgt, was nach der Nernst'schen Potentialgleichung für eine Zwei-Elektronen-Reaktion zu erwarten wäre.

Besonders bevorzugt besteht die lipophile Grundmembran aus PVC oder Silikonkautschuk. Es hat sich gezeigt, daß bei Verwendung einer solchen Grundmembran eine Verdoppelung des Nernst-Potentials auftritt.

Bei der Oxidation von $H_2O_2$ nach der Reak-

tionsgleichung:

$$H_2O_2 - 2e^- \longrightarrow O_2 + 2H^+$$

entsteht deshalb eine EMK von 59 mV/Dekade (bei 25° C).

Offenbar können sich unter diesen Bedingungen zwei stabile, serielle Potentialanteile ausbilden. Dies bewirkt einen Signalgewinn von 100 %.

Besonders bevorzugt werden Grundmembranen mit geringer Membranstärke, insbesondere solche mit einer Membranstärke von 10-50 $\mu$m verwendet.

Nachfolgend wird die Erfindung anhand einer bevorzugten Ausführungsform unter Bezugnahme auf die beigefügten Zeichnungen noch näher erläutert.

Es zeigen:

Fig. 1 eine schematische Darstellung einer bevorzugten Ausführungsform der Erfindung im Schnitt;

Fig. 2 eine schematische Darstellung eines Teils der Ausführungsform nach Fig. 1 zur Verdeutlichung einer Weiterbildung gemäß der Erfindung.

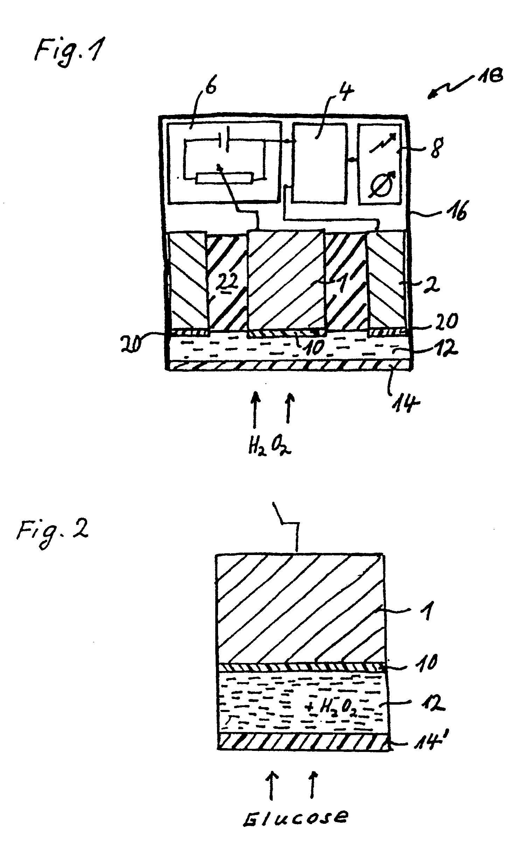

In Fig. 1 ist eine Meßelektrode 1, vorzugsweise aus Gold, die aber auch aus Platin sein kann, und eine vorzugsweise ringförmig angeordnete Referenzelektrode 2, über einen Meßverstärker 4 mit einem Eingangswiderstand von $10^{12}\Omega$ (für den Fall der Potentialmessung) bzw. einen Meßwiderstand von $10^6\Omega$ (für den Fall der amperometrischen Messung), mit dem Plus- bzw. Minuspol einer Polarisationsspannungsquelle 6 verbunden. Der durch das Meßwerk 4 gemessene Spannungs- oder Stromwert wird an ein Anzeigegerät 8 gegeben, das auch, wie symbolisch angedeutet, mit einer Fernanzeige, bspw. einem Sender, versehen ist. Dieser Sender sendet den Meßwert bspw. an einen (nicht gezeigten) Mikroprozessor, der daraus unter Berücksichtigung von Einzelheiten der Meßbedingungen die Konzentration des zu messenden $H_2O_2$ errechnet und den errechneten Wert ggfs. zum Erstellen weiterer Werte benutzt, wie noch ausgeführt werden wird. Eine Schutzmembran 10 aus einem lipophilen Material, vorzugsweise PVC, schließt die Meßelektrode (Anode) 1 gegen den im Elektrolytraum enthaltenen Elektrolyten 12 ab. Die Membran 10 ist durch Beimischung eines geeigneten Salzes mit lipophilen Anionen versetzt worden. Als Salz hat sich, wie erwähnt, Hexa-Decyl-Pyridinium-Chlorid bewährt. Sie ist ferner $H^+$-undurchlässig. In der Membran enthaltene carriergebundene Ionen befinden sich auch im Elektrolyten 12.

Die Referenzelektrode 2 ist vorzugsweise eine Ag/AgCl-Elektrode oder eine Kalomelelektrode. Sie kann ebenfalls mit einer schützenden Referenzelektrodenmembran versehen sein, die aber auch entfallen kann. Der Elektrolyt 12 bedeckt die geschilderte Elektrodenanordnung. Der Elektrolytraum ist im übrigen im wesentlichen an der von den Elektroden abgewandten Seite durch eine $H_2O_2$-durchlässige, wasserundurchlässige Membran 14, z.B. 25-100$\mu$ Polytetrafluoräthylen, abgeschlossen. Grenzt diese Membran an ein $H_2O_2$ enthaltendes Medium, so bildet sich durch Diffusion ein Gleichgewicht zwischen dem $H_2O_2$ im Außenraum und dem Elektrolyten. Das $H_2O_2$ im Elektrolyten gelangt teilweise durch die Membran 10 an die Meßelektrode und wird dort oxidiert. Das dabei entstehende Potential bzw. der dabei entstehende Strom wird gemessen und liefert ein Maß für die $H_2O_2$-Konzentration. Zwischen den Elektroden 1 und 2 befindet sich ein isolierendes, inertes Material 22.

Die ganze Anordnung kann, bis auf die Außenfläche der Membran 14, verkapselt werden, was mit der anhand der Fig. 2 zu schildernden Ausführungsform besonders vorteilhaft ist.

In Fig. 2 ist die Meßelektrode 1 wieder mit der oben geschilderten lipophilen PVC-Membran 10 gegen den Elektrolyten 12 abgedeckt. Im Elektrolyten befindet sich ein Enzym, durch das mittels einer Enzymreaktion aus einer zugehörigen Substanz ein Reaktionsprodukt plus $H_2O_2$ erzeugt wird. Die (Abschluß-)Membran 14' ist dabei als Porenmembran so ausgebildet, daß die Substanz, z.B. Glucose, durch die Membran hindurchdiffundieren kann und sich im Elektrolyten ebenfalls eine Gleichgewichtskonzentration der Substanz einstellt. Diese Substanz wird in der Enzymreaktion umgewandelt und das in dieser Ausführungsform erst im Elektrolytraum entstehende $H_2O_2$ ebenso wie im Ausführungsbeispiel der Fig. 1 gemessen. Diese Messung erlaubt wiederum Rückschlüsse auf die Menge der vorhandenen Substanz. Dabei ist wichtig, daß das neben $H_2O_2$ entstehende Endprodukt, im Falle von Glucose und dem Enzym Glucose-Oxidase Gluconsäurelacton, durch die Membran 14' hinausdiffundieren kann, um eine dauernde Anreicherung im Elektrolytraum und damit auch eine Verschiebung des Reaktionsgleichgewichts zu vermeiden. Hingegen muß die Membran 14' für die Enzymmoleküle undurchlässig sein, um Verfälschungen des Meßergebnisses zu verhindern.

Mit der Weiterbildung gemäß Fig. 2 versehen kann die verkapselte Gesamtanordnung nach Fig. 1 implantiert werden und über längere Zeiträume z.B. die Glucosekonzentration messen. Die Meßergebnisse werden entweder fortlaufend oder ggfs. auf äußeren Abruf über einen mitverkapselten Signalempfänger, abgegeben, bspw. an einen Mikroprozessor. Dieser stellt fest, ob der zuträgliche Bereich der Glucosekonzentration über- oder unterschritten ist. Es können dann z.B. Warnsignale abgegeben werden oder konkrete Maßnahmen (Menge aufzunehmender Nahrungsmittel) angegeben werden, um das Ungleichgewicht zu beheben. Ferner kann eine Insulinpumpe angesteuert werden. Mit der Anordnung kann auch in bekannter Weise amperometrisch gemessen werden.

## Ansprüche

**1.** Anordnung zum Messen der Konzentration eines durch inerte Membranen diffusiblen Stoffes, der zu Redoxreaktionen fähig ist, mit
einer Meßelektrode (1) aus Metall, vorzugsweise Edelmetall, mit einer die Meßelektrode (1) teilweise bedeckenden, lipophilen Membran (10), die zwischen der Meßelektrode und einem in einem Elektrolytraum enthaltenen Elektrolyten (12) angeordnet ist,
und einer Referenzelektrode (2),
wobei der zu messende Stoff im Elektrolytraum vorliegt,
**dadurch gekennzeichnet,**
daß der Stoff $H_2O_2$ ist,
daß die Gesamtmembran (10) lipophile Ionen und/oder, in an sich bekannter Weise, carriergebundene Ionen enthält, daß die Grundmembran (10) protonenimpermeabel ist, und daß die Meßelektrode als Anode ausgebildet ist.

**2.** Anordnung nach Anspruch 1, dadurch gekennzeichnet, daß die Ionen Anionen sind.

**3.** Anordnung nach Anspruch 1 oder 2, dadurch gekennzeichnet, daß die carriergebundenen Ionen Kationen, insbesondere Kaliumionen, sind.

**4.** Anordnung nach einem der Ansprüche 1-3, dadurch gekennzeichnet, daß der Carrier Valinomycin ist.

**5.** Anordnung nach Anspruch 1, dadurch gekennzeichnet, daß der Carrier ein Kationencarrier von hoher Mobilität und Spezifität, besonders für Protonen, bevorzugt Tri-n-dodecylamin, ist.

**6.** Anordnung nach einem der vorhergehenden Ansprüche, dadurch gekennzeichnet, daß sie eine Polarisationsspannungsquelle (6) aufweist, deren Pluspol an die Meßelektrode (1) angeschlossen ist.

**7.** Anordnung nach einem der vorhergehenden Ansprüche, dadurch gekennzeichnet, daß der Elektrolytraum durch eine Abschlußmembran (14) gegen den Außenraum abgeschlossen ist.

**8.** Anordnung nach Anspruch 7, dadurch gekennzeichnet, daß die Abschlußmembran (14) für $H_2O_2$ durchlässig ist.

**9.** Anordnung nach Anspruch 7, daduroh gekennzeichnet, daß der Elektrolytraum auch als Reaktionsraum ausgebildet ist, in dem sich ein Enzym befindet, das eine durch die Abschlußmembran (14) in den Elektrolyten (12) diffusible Substanz unter Bildung von $H_2O_2$ umsetzt.

**10.** Anordnung nach Anspruch 9, dadurch gekennzeichnet, daß das Enzym in wässriger Lösung oder strukturgebunden vorliegt.

**11.** Anordnung nach Anspruch 9 oder 10, dadurch gekennzeichnet, daß die Abschlußmembran (14 für kleinere Moleküle durchlässig, für größere Moleküle hingegen undurchlässig ist.

**12.** Anordnung nach einem der Ansprüche 9-11, dadurch gekennzeichnet, daß das Enzym Glucose-Oxidase ist und die Abschlußmembran (14) für Glucose und Gluconsäurelacton durchlässig ist.

**13.** Anordnung nach einem der Ansprüche 9-12, dadurch gekennzeichnet, daß die Anordnung, ggfs. einschließlich der Polarisationsspannungsquelle (6), als, integrierte, implantierbare Einheit (18) ausgebildet ist und einen Geber oder Sender (8) für das Meßergebnis aufweist.

**14.** Anordnung nach einem der vorhergehenden Ansprüche, dadurch gekennzeichnet, daß die Bezugselektrode (2) eine Edelmetallelektrode ist, die als Kathode ausgebildet ist, und die mit einer Membran (20) bedeckt ist, die vorzugsweise einen Protonencarrier, besonders bevorzugt Tri-n-dodecylamin, enthält.

**15.** Anordnung nach einem der vorhergenden Ansprüche, dadurch gekennzeichnat, daß die Grundmembran aus PVC oder Silikonkautschuk besteht.

## Claims

**1.** Arrangement for measuring the concentration of a substance diffusable through inert membranes and capable of redox reactions, having a measuring electrode (1) of metal, preferably noble metal, with a lipophilous membrane (10) partially covering the measuring electrode (1 ) and located between the measuring electrode and an electrolyte (12) contained in an electrolyte chamber, and a reference electrode (2), wherein the substance to be measured is in the electrolyte chamber
**characterized in**
that the substance is $H_2O_2$,
that the combined membrane (10) contains lipophilous ions and/or, in a manner known per se, carrier bound ions,
that the base membrane (10) is proton impermeable, and
that the measuring electrode is formed as anode.

2. Arrangement according to claim 1, characterized in
that the ions are anions.

3. Arrangement according to claim 1 or 2, characterized in
that the carrier-bound ions are cations, especially potassium ions.

4. Arrangement according to claim 1, characterized in
that the carrier is valinomycin.

5. Arrangement according to claim 1, characterized in
that the carrier is a cation carrier of high mobility and specificity, especially for protons, preferredly tri-n-dodecyl-amine.

6. Arrangement according to one of the preceeding claims, characterized in
that it comprises a polarization voltage source (6) the plus pole of which is connected to the measuring electrode (1).

7. Arrangement according to one of the preceeding claims, characterized in
that the electrolyte chamber is separated from the outside by a closure membrane (14).

8. Arrangement according to claim 7, characterized in
that the closure membrane (14) is permeable for $H_2O_2$.

9. Arrangement according to claim 7, characterized in
that the electrolyte chamber is also formed as a reaction chamber which contains an enzyme converting, while forming $H_2O_2$, a substance diffusable through the closure membrane (14) into the electrolyte (12).

10. Arrangement according to claim 9, characterized in
that the enzyme is in an aqueous solution or structure-bound.

11. Arrangement according to claim 9 or 10, characterized in
that the closure membrane (14) is permeable for smaller molecules, whereas impermeable for larger molecules.

12. Arrangement according to one of claims 9 - 11, characterized in
that the enzyme is glucose-oxidase and the closure membrane (14) is permeable for glucose and glucon-acid-lactone.

13. Arrangement according to one of claims 9 - 12, characterized in
that the arrangement, if possible and desired including the polarization voltage source (6), is formed as an integrated, implantable unit (18) and comprises a transmitter or sender (8) for the measurement result.

14. Arrangement according to one of the preceeding claims, characterized in
that the reference electrode (2) is a noble metal electrode which is formed as cathode and which is covered with a membrane (20) which comprises preferably a proton carrier, especially preferredly tri-n-dodecyl-amine.

15. Arrangement according to one of the preceeding claims, characterized in
that the base membrane is composed of polyvinylchloride or silicon rubber.

**Revendications**

1. Appareillage pour la mesure de la concentration d'une substance pouvant diffuser au travers de membranes inertes et susceptible de réactions redox, comprenant
une électrode de mesure (1) en métal, de façon préférée un métal précieux, comportant une membrane lipophile (10) qui recouvre partiellement l'électrode de mesure (1), et qui est disposée entre l'électrode de mesure et un électrolyte (12) contenu dans une chambre d'électrolyte,
et une électrode de référence (2),
la substance à mesurer étant, là, présente dans la chambre d'électrolyte,
**caractérisé en ce que**
la substance est $H_2O_2$,
en ce que la membrane (10) dans sa totalité renferme des ions lipophiles et/ou de manière connue en soi, des ions combinés à un véhiculeur,

en ce que la membrane de base (10) est imperméable aux protons, et
en ce que l'électrode de mesure est réalisée sous forme d'anode.

2. Appareillage selon la revendication 1, caractérisé en ce que
les ions sont des anions.

3. Appareillage selon la revendication 1 ou 2, caractérisé en ce que
les ions combinés à un véhiculeur, sont des cations, notamment des ions potassium.

4. Appareillage selon l'une des revendications 1 à 3, caractérisé en ce que
le véhiculeur est de la valinomycine.

5. Appareillage selon la revendication 1, caractérisé en ce que
le véhiculeur est un véhiculeur de cations de grande mobilité et spécificité, notamment pour des protons, et est, de préférence, de la tri-n-dodécylamine.

6. Appareillage selon l'une des revendications précédentes, caractérisé en ce
qu'il présente une source de tension de polarisation (6) dont le pôle positif est raccordé à l'électrode de mesure (1).

7. Appareillage selon l'une des revendications précédentes, caractérisé en ce que
la chambre d'électrolyte est isolée de l'espace extérieur par une membrane de fermeture (14).

8. Appareillage selon la revendication 7, caractérisé en ce que
la membrane de fermeture (14) est perméable à $H_2O_2$.

9. Appareillage selon la revendication 7, caractérisé en ce que
la chambre d'électrolyte est également réalisée sous la forme d'une chambre de réaction dans laquelle se trouve une enzyme qui convertit, en formant de l'$H_2O_2$, une substance pouvant diffuser dans l'électrolyte au travers de la membrane de fermeture (14).

10. Appareillage selon la revendication 9, caractérisé en ce que
l'enzyme est présente en solution aqueuse ou en étant combiné à la structure.

11. Appareillage selon la revendication 9 ou 10, caractérisé en ce que
la membrane de fermeture (14) est perméable aux petites molécules, alors qu'elle est par contre imperméable aux molécules plus grandes.

12. Appareillage selon l'une des revendications 9 à 11, caractérisé en ce que
l'enzyme est de l'oxydase de glucose et que la membrane de fermeture (14) est perméable au glucose et à la lactone d'acide gluconique.

13. Appareillage selon l'une des revendications 9 à 12, caractérisé en ce que
l'appareillage, y compris, éventuellement, la source de tension de polarisation (6), est réalisé sous la forme d'une unité intégrée (18) pouvant être implantée, et présente un transmetteur ou émetteur (8) pour le résultat de mesure.

14. Appareillage selon l'une des revendications précédentes, caractérisé en ce que
l'électrode de référence (2) est une électrode en métal précieux, qui est réalisée sous la forme de cathode, et qui est recouverte d'une membrane (20), qui, de préférence, renferme un véhiculeur de protons, de manière particulièrement préférée de la tri-n-dodécyl-amine.

15. Appareillage selon l'une des revendications précédentes, caractérisé en ce que
la membrane de base est réalisée en PVC ou en caoutchouc silicone.

Fig. 1

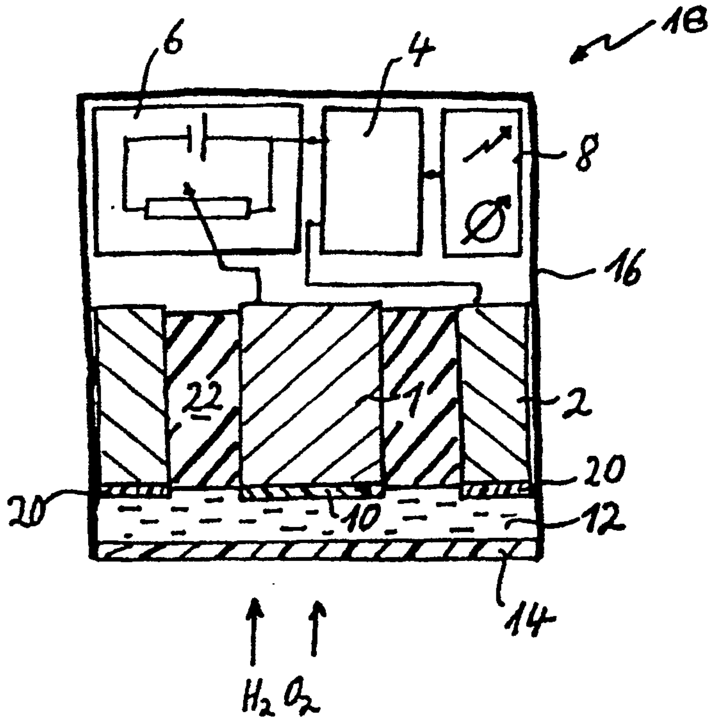


Fig. 2